# EUROPEAN PATENT APPLICATION

(11) **EP 4 818 760 A1**
(43) Date of publication of application: **07.10.2026**
(21) Application number: 26163828.2
(22) Date of filing: 11.03.2026
(51) Int. Cl.: G01T 1/29

(54) **RADIATION DETECTION DEVICE WITH AIR COOLING ARRANGEMENT AND CORRESPONDING CT APPARATUS**

(30) Priority: 27.03.2025 JP 2025054301
(71) Applicant: FUJIFILM Corporation, Tokyo 106-8620 (JP)
(72) Inventor: HAYASHI, Tatsuhiko, Tokyo, 106-8620 (JP); KASAMA, Minoru, Tokyo, 106-8620 (JP)
(74) Representative: Dehns Germany Partnerschaft mbB

(57) **Abstract**

Provided are a radiation detection device and a CT apparatus capable of efficiently performing cooling while suppressing complexity of a structure.

A radiation detection device according to the present disclosure is divided into a plurality of compartments and includes, in each of the plurality of compartments, a detector module that detects radiation, a heat sink that is attached to the detector module, an intake port and an exhaust port that are disposed on the same side with respect to the heat sink, a separation plate that is provided between the intake port and the exhaust port and separates intake and exhaust, and an exhaust fan that is attached to the exhaust port, in which the heat sink has a plurality of fins that extend along a flow path of air exhausted from the exhaust port.

## Description

### BACKGROUND OF THE INVENTION

### 1. Field of the Invention

The technology of the present disclosure relates to a radiation detection device and a CT apparatus.

### 2. Description of the Related Art

In a computed tomography (CT) apparatus, temperature control of a radiation detection device that detects radiation, which has been emitted from a radiation source and transmitted through a subject, is important for maintaining performance and reliability of the apparatus. An air cooling method using a fan is generally used as the temperature control of the radiation detection device. For example, the following are known: a method for dissipating heat of a radiation detection device using an exhaust fan that operates at a constant rotation speed; and a method for maintaining the temperature of a radiation detection device constant while using a heater in combination.

In particular, in a photon counting computed tomography (PCCT) apparatus, which is a type of CT apparatus, photon counting is performed in each of a plurality of detector modules constituting a radiation detection device. Therefore, the amount of heat generated is large, and temperature variation for each detector module is large, as compared to the CT apparatus according to the related art. In the PCCT apparatus, heat is generated in a process in which incident photons are converted into electric charges by a semiconductor layer and the electric charges are counted by a photon counting circuit, and the amount of heat generated varies depending on a counting rate. Therefore, in the above-described temperature control, it is difficult to maintain the radiation detection device at an appropriate temperature.

Therefore, a technique has been proposed that performs temperature control for each detector module in a PCCT apparatus (for example, see JP2024-037489A). The apparatus disclosed in JP2024-037489A includes an acquisition unit that acquires information related to the amount of heat generated from the detector module, blowing units that are installed in an intake port and an exhaust port of a housing, an opening and closing mechanism that is disposed between the intake port or the exhaust port of the housing and the detector module, and an opening and closing controller that controls the opening and closing mechanism based on heat generation information. As described above, JP2024-037489A proposes a method that controls the opening and closing mechanism based on the heat generation information for each detector module to adjust the amount of air blown, thereby maintaining the temperature of the radiation detection device constant.

In addition, a method is known in which a heat sink is used to cool a heat generating body, such as a radiation detector, in a CT apparatus or the like (for example, see JP2024-078759A). The apparatus disclosed in JP2024-078759A has an intake port and an exhaust port, accommodates a heat sink in a duct that is adhered to the heat generating body, and exhausts air in the duct using a fan provided on one of the intake port or the exhaust port.

### SUMMARY OF THE INVENTION

The technique disclosed in JP2024-037489A has the following problems. First, it is difficult to control the temperature of the detector module according to a change in the temperature of the surroundings using only the information related to the amount of heat generated from the detector module.

Second, a plurality of detector modules are provided in the radiation detection device, and it may be difficult to dispose the opening and closing mechanism between the intake port or the exhaust port and the detector module from the viewpoint of space saving of the apparatus. Further, the addition of the opening and closing mechanism also involves the disadvantage of increasing the cost of the apparatus.

Third, another problem is that, since a turntable, a frame, and a slip ring are disposed on a rear surface of the radiation detection device, the amount of intake and exhaust air decreases and cooling efficiency is reduced in a case where a portion of an intake and exhaust structure is disposed on the rear surface side of the radiation detection device.

In the technique disclosed in JP2024-078759A, the intake port is disposed at one end of the heat sink in a direction in which air flows, and the exhaust port is disposed at the other end. Therefore, in a case where the cooling technique disclosed in JP2024-078759A is applied to the apparatus disclosed in JP2024-037489A, the same problem as described above occurs. For example, since the intake port or the exhaust port is located on the rear surface side of the radiation detection device, there is a problem that the amount of intake and exhaust air is reduced by the structure on the rear surface side, resulting in a reduction in cooling efficiency.

Furthermore, in recent years, a cooling method using a water cooling technique has also been proposed. However, the method has not been widely put into practical use because of high costs and complicated structures.

Therefore, the technology according to the present disclosure provides a radiation detection device and a CT apparatus capable of efficiently performing cooling while suppressing complexity of a structure.

According to an aspect of the present invention, there is provided a radiation detection device that is divided into a plurality of compartments, the radiation detection device comprising in each of the plurality of compartments: a detector module that detects radiation; a heat sink that is attached to the detector module; an intake port and an exhaust port that are disposed on the same side with respect to the heat sink; a separation plate that is provided between the intake port and the exhaust port and separates intake and exhaust; and an exhaust fan that is attached to the exhaust port, in which the heat sink has a plurality of fins that extend along a flow path of air exhausted from the exhaust port.

According to another aspect of the present invention, there is provided a CT apparatus comprising: a radiation source that emits radiation; and a radiation detection device that is divided into a plurality of compartments, in which the radiation source and the radiation detection device are configured to be rotatable about a central axis in a state in which the radiation source and the radiation detection device face each other, the CT apparatus includes, in each of the plurality of compartments, a detector module that detects the radiation, a heat sink that is attached to the detector module, an intake port and an exhaust port that are disposed on the same side with respect to the heat sink, a separation plate that is provided between the intake port and the exhaust port and separates intake and exhaust, and an exhaust fan that is attached to the exhaust port, and the heat sink has a plurality of fins that extend along a flow path of air exhausted from the exhaust port.

The plurality of fins may form a groove in a direction along the flow path.

The intake port and the exhaust port may be formed in a front cover that constitutes a front surface side of each of the plurality of compartments.

The intake port and the exhaust port may be disposed at different distances from the central axis.

The intake port may be disposed at a position that is farther from the central axis than the exhaust port.

The plurality of compartments may be partitioned by a plurality of partition plates.

An intake cover that covers a periphery of the intake port and an exhaust cover that covers a periphery of the exhaust port may be provided in each of the plurality of compartments, and intake and exhaust may be separated from each other outside the compartment by the intake cover and the exhaust cover.

An opening may be formed in the intake cover on a side of a rotation direction in which the radiation source and the radiation detection device are rotated.

Lengths of a plurality of the intake covers in a direction of the central axis may be reduced stepwise toward the rotation direction.

An opening may be formed in the exhaust cover on a side opposite to the rotation direction in which the radiation source and the radiation detection device are rotated.

Lengths of a plurality of the exhaust covers in a direction of the central axis may be reduced stepwise toward the side opposite to the rotation direction.

The intake port may have a lattice-like opening shape.

A plurality of through-holes and a shielding plate that shields the radiation may be provided in a bottom portion of each of the plurality of compartments, and the shielding plate may be disposed in a region that is irradiated with the radiation transmitted through the detector module.

A sound absorbing sheet may be attached to at least one of the intake cover or the exhaust cover.

The CT apparatus according to the present disclosure may comprise a temperature measurement device that measures a temperature of the air exhausted from the exhaust port; and a control substrate that controls a rotation speed of the exhaust fan based on the temperature.

The temperature measurement device may be disposed between the detector module and the exhaust fan.

The control substrate may control the rotation speed of the exhaust fan based on a rotation speed of the radiation source and the radiation detection device, in addition to the temperature.

The detector module may include a circuit element including a photon counting circuit that counts the number of photons, and the temperature measurement device may be incorporated into the circuit element.

The detector module may include a plurality of the circuit elements, the temperature measurement device may be incorporated into each of the plurality of circuit elements, and the control substrate may control the rotation speed based on an average value or a median value of a plurality of measured temperature values.

According to the technology of the present disclosure, it is possible to provide a radiation detection device and a CT apparatus capable of efficiently performing cooling while suppressing complexity of a structure.

### BRIEF DESCRIPTION OF THE DRAWINGS

FIG. 1 is a diagram schematically showing a configuration of a CT apparatus according to an embodiment.
FIG. 2 is a diagram schematically showing a configuration of a gantry.
FIG. 3 is a diagram showing an internal configuration of an X-ray detection device.
FIG. 4 is a diagram showing the internal configuration of the X-ray detection device.
FIG. 5 is a diagram showing a configuration of a detector module.
FIG. 6 is a diagram showing the appearance of the X-ray detection device.
FIG. 7 is a diagram showing a configuration of internal compartments of the X-ray detection device.
FIG. 8 is a diagram showing a detailed configuration of the internal compartments of the X-ray detection device.
FIG. 9 is a diagram showing a configuration of an intake port.
FIG. 10 is a diagram showing a bottom structure of the X-ray detection device.
FIG. 11 is a flowchart showing a flow of temperature control by a control substrate.
FIG. 12 is a diagram showing a modification example of an exhaust cover.
FIG. 13 is a diagram showing a modification example of an intake cover.
FIG. 14 is a diagram showing a modification example in which a length of the intake cover in a Z direction is changed.
FIG. 15 is a diagram showing a modification example in which a position of an opening of the exhaust cover is changed.
FIG. 16 is a diagram showing a modification example in which the length of the exhaust cover in the Z direction is changed.
FIG. 17 is a flowchart showing a modification example of the temperature control.

### DESCRIPTION OF THE PREFERRED EMBODIMENTS

Hereinafter, embodiments according to the technology of the present disclosure will be described with reference to the drawings. A CT apparatus according to an embodiment of the present disclosure is applied to a PCCT-type CT apparatus that detects radiation emitted from a radiation source and generates a radiographic image based on an electric signal corresponding to the number of photons of the radiation. In the present embodiment, a case where the radiation is X-rays will be described as an example.

### Embodiment

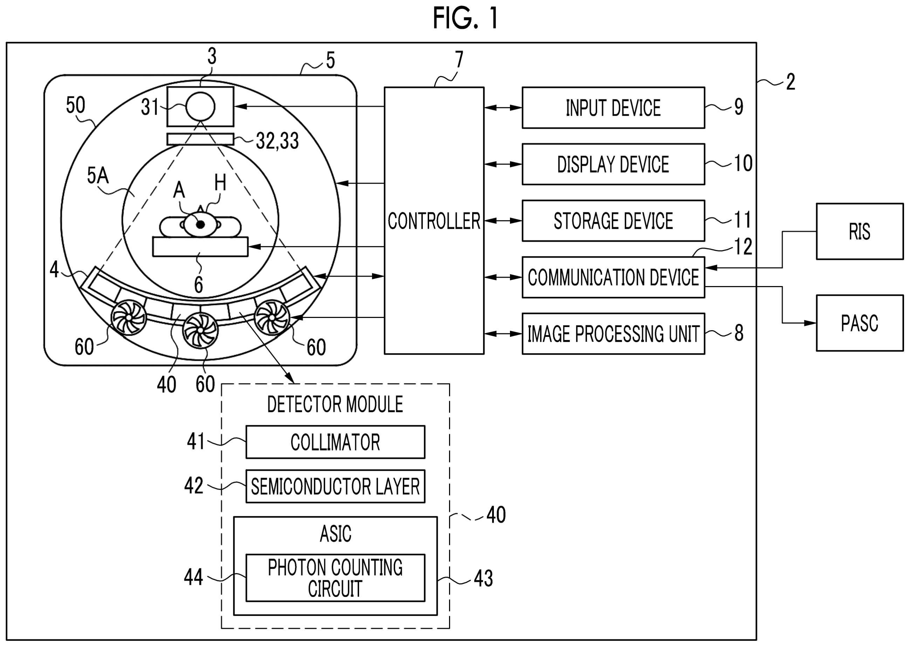

FIG. 1 schematically shows a configuration of a CT apparatus 2 according to an embodiment. The CT apparatus 2 includes an X-ray source 3, an X-ray detection device 4, a gantry 5, a bed 6, a controller 7, and an image processing unit 8. A circular opening portion 5A for disposing the bed 6 on which a subject H is placed is provided at the center of the gantry 5. In addition, the gantry 5 is provided with a rotating plate 50 that is fixed at a position where the X-ray source 3 and the X-ray detection device 4 face each other and a drive mechanism (not shown) for rotating the rotating plate 50 about a central axis A. Further, a plurality of exhaust fans 60 are provided in the X-ray detection device 4. The X-ray source 3 is an example of a "radiation source" according to the technology of the present disclosure. The X-ray detection device 4 is an example of a "radiation detection device" according to the technology of the present disclosure.

In addition, hereinafter, a mechanism that includes the X-ray source 3, the X-ray detection device 4, and the rotating plate 50 and performs imaging while being rotated is referred to as a "scanner".

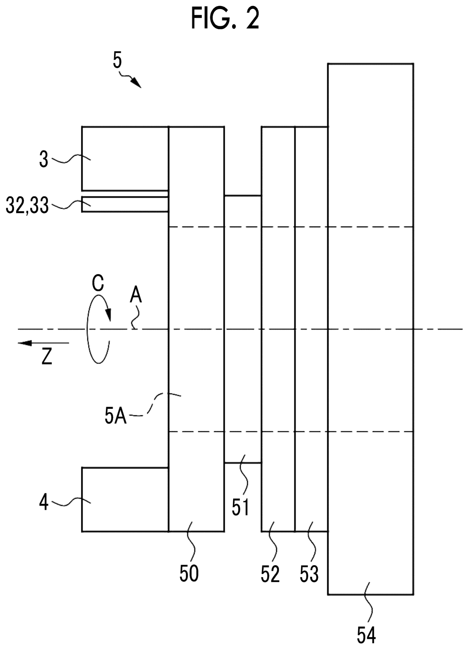

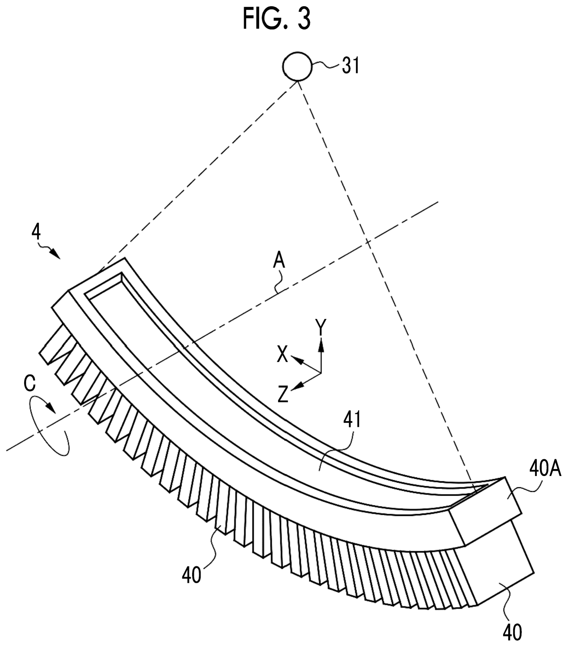

Hereinafter, in the present disclosure, a circumferential direction of the opening portion 5A is referred to as an X direction, a radial direction is referred to as a Y direction, and a direction parallel to the central axis A is referred to as a Z direction (see FIGS. 2 and 3). The Z direction is orthogonal to the X direction and the Y direction and is generally a body axis direction of the subject H. In addition, a direction in which the rotating plate 50 is rotated about the central axis A is referred to as a "rotation direction C".

The X-ray source 3 includes an X-ray tube 31. In addition, an X-ray filter 32 and a bowtie filter 33 are provided on the emission side of the X-ray source 3. The X-ray tube 31 generates X-rays and irradiates the subject H with the generated X-rays. The X-ray filter 32 adjusts the dose of the X-rays emitted from the X-ray tube 31. The bowtie filter 33 optimizes an exposure dose by increasing the dose near the center and reducing the dose around the periphery to suppress the exposure dose in a peripheral portion.

The controller 7 is configured by a processor such as a central processing unit (CPU). The controller 7 controls the operations of the X-ray source 3, the X-ray detection device 4, the gantry 5, and the bed 6. Specifically, the controller 7 controls the emission of the X-rays from the X-ray tube 31 of the X-ray source 3, the detection of the X-rays by the X-ray detection device 4, the rotation of the rotating plate 50 of the gantry 5, and the movement of the bed 6. In addition, the controller 7 acquires data output from the X-ray detection device 4.

The image processing unit 8 is an image processing processor that performs a reconstruction process based on the data acquired from the X-ray detection device 4 by the controller 7 to generate a tomographic image. The image processing unit 8 may be configured as a portion of the controller 7.

In addition, an input device 9, a display device 10, a storage device 11, and a communication device 12 are connected to the controller 7. The input device 9 is a device for an operator to input an operation instruction and is configured by a keyboard, a mouse, and the like. The display device 10 is a display, such as a liquid crystal display, and displays an operation screen, a tomographic image, and the like. The storage device 11 is a memory, a storage device, or the like and stores a tomographic image, a program, various types of information, and the like.

The communication device 12 is a communication interface for communication with radiology information systems (RIS), picture archiving and communication systems (PACS), and the like. The communication device 12 performs transmission control according to a communication protocol based on a wired or wireless communication standard.

FIG. 2 schematically shows a configuration of the gantry 5. The gantry 5 includes the rotating plate 50, a bearing 51, a frame 52, a slip ring 53, and a fixing portion 54. The bearing 51, the frame 52, the slip ring 53, and the fixing portion 54 are disposed in this order on a side of the rotating plate 50 that is opposite to the X-ray detection device 4.

The bearing 51 is provided between the rotating plate 50 and the frame 52 and smoothly rotates the rotating plate 50. The slip ring 53 is a member for transmitting power or signals between the rotating plate 50 that is rotated and the fixing portion 54, and the structure of the slip ring 53 consists of a conductive ring and a brush that are disposed concentrically. The slip ring 53 is used to supply power to the X-ray detection device 4 and the X-ray tube 31 and to transmit the data output from the X-ray detection device 4 to the controller 7.

The fixing portion 54 is a fixing structure that supports the frame 52 and the slip ring 53, and has a contact portion that comes into contact with the conductive brush of the slip ring 53 such that power and data are transmitted.

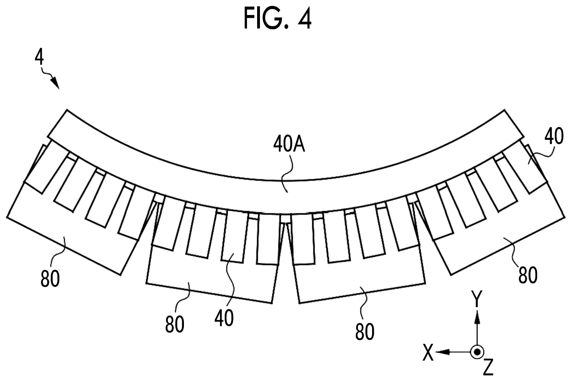

FIGS. 3 and 4 show an internal configuration of the X-ray detection device 4. The X-ray detection device 4 includes a plurality of detector modules 40, a plurality of control substrates 80, and a case 40A. The case 40A has an arc shape that has a focal point of the X-ray tube 31 as the center and holds a plurality of detector modules 40. The plurality of detector modules 40 are arranged in a channel direction which is a direction along the arc shape. For example, the number of detector modules 40 is about 30 to 50. The plurality of control substrates 80 are disposed on the rear surface side of the plurality of detector modules 40 and are connected to the plurality of detector modules 40 by cables (not shown). Each of the control substrates 80 has a control circuit.

Each of the detector modules 40 includes a collimator 41, a semiconductor layer 42, and an application specific integrated circuit (ASIC) 43 (see FIG. 1).

The collimator 41 is disposed on an X-ray incident side of the semiconductor layer 42 and is fixed to a frame 45 of the detector module 40. In addition, the collimator 41 may be fixed to the case 40A of the X-ray detection device 4. The collimator 41 limits an incident direction of the X-rays onto the semiconductor layer 42 to remove scattered rays. The semiconductor layer 42 is made of cadmium zinc telluride (CZT), cadmium telluride (CdTe), or the like, converts the incident X-rays that have been transmitted through the subject H into charges corresponding to photons, and outputs the charge.

The ASIC 43 is disposed on a side of the semiconductor layer 42 that is opposite to the collimator 41. The ASIC 43 is an example of a circuit element including a photon counting circuit 44. The photon counting circuit 44 counts the number of charges output from the semiconductor layer 42 as the number of photons and outputs a count signal. For example, the photon counting circuit 44 is configured to include an amplifier circuit, a waveform shaping circuit, a comparator circuit, a counter circuit, and the like. The photon counting circuit 44 may include an analog-to-digital (A/D) converter.

Further, electrodes for applying a high voltage to the semiconductor layer 42 are formed on an upper surface and a lower surface of the semiconductor layer 42. A plurality of pixels are configured in the semiconductor layer 42 by patterning the electrode on the lower surface side of the semiconductor layer 42. The photon counting circuit 44 counts photons for each pixel and outputs the count signal. This count signal is output as the above-described data from the X-ray detection device 4 through a circuit substrate 46 that will be described below.

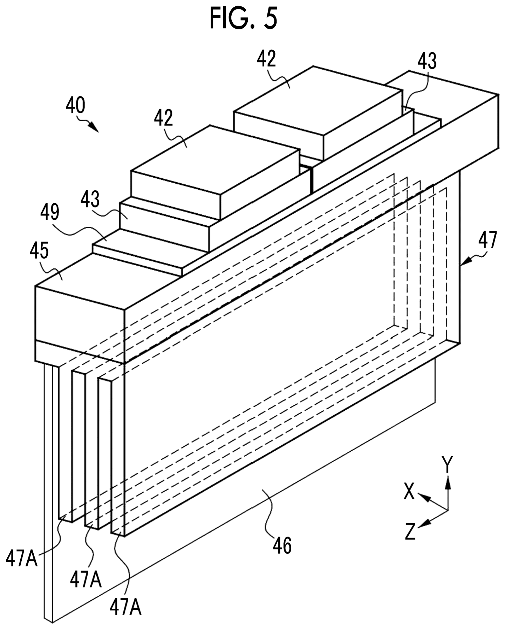

FIG. 5 shows a configuration of the detector module 40. The detector module 40 includes a plurality of semiconductor layers 42, a plurality of ASICs 43, a substrate 49, the frame 45, the circuit substrate 46, and a heat sink 47. The frame 45 extends in a column direction (corresponding to the Z direction) orthogonal to the channel direction. The frame 45 is made of metal such as aluminum (Al) or copper (Cu). Both end portions of the frame 45 in the column direction are fixed to the case 40A. The substrate 49 is made of, for example, ceramic and is provided on the frame 45.

The plurality of ASICs 43 are arranged in the column direction on an upper surface of the substrate 49. In the present embodiment, two ASICs 43 are arranged on the substrate 49. However, the number of ASICs 43 is not limited to this example. In addition, the plurality of the ASICs 43 may be arranged on the frame 45 in a plurality of columns. The semiconductor layer 42 is disposed on each ASIC 43.

The circuit substrate 46 is connected to the substrate 49 through a cable (for example, a flexible cable) (not shown). The count signal output from each ASIC 43 is transmitted as the above-described data to the controller 7 through the substrate 49, the circuit substrate 46, and the control substrate 80.

The heat sink 47 is a member for efficiently dissipating heat generated in the ASIC 43 and is connected to a lower surface of the frame 45. The heat sink 47 is made of metal such as aluminum (Al). In the present embodiment, the heat sink 47 is a plate-fin-type heat sink configured by arranging flat plate-shaped fins 47A in parallel. In the present embodiment, the heat sink 47 has a rectangular shape that is long in the Z direction.

A plurality of fins 47A are arranged in the X direction at a constant arrangement pitch (that is, an equal pitch) or an unequal pitch. A groove that extends in the Z direction is configured by two adjacent fins 47A. In the present embodiment, three fins 47A are arranged. However, the number of fins 47A is not limited to this example.

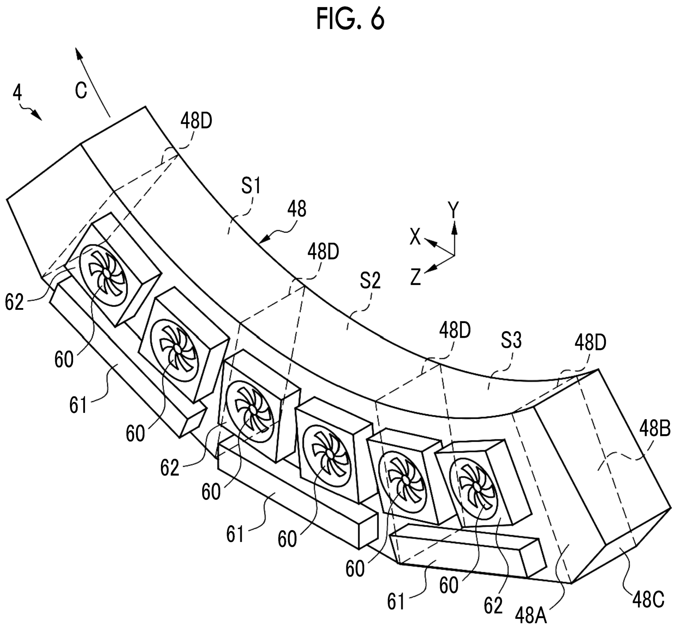

FIG. 6 shows the appearance of the X-ray detection device 4. The plurality of detector modules 40 are accommodated in a housing 48. The housing 48 includes a front cover 48A, a rear block 48B, and a bottom cover 48C. The front cover 48A and the rear block 48B face each other in the Z direction. One end of the bottom cover 48C in the Z direction is connected to the front cover 48A, and the other end of the bottom cover 48C is connected to the rear block 48B.

In addition, the inside of the housing 48 is divided into a plurality of compartments by a plurality of partition plates 48D in the channel direction. One end of the partition plate 48D in the Z direction is connected to the front cover 48A, and the other end of the partition plate 48D is connected to the rear block 48B. In the present embodiment, the housing 48 includes three compartments S1 to S3. Hereinafter, in a case where the compartments S1 to S3 are not distinguished from each other, the compartments S1 to S3 are simply referred to as compartments S. The number of compartments S is not limited to three. The front cover 48A constitutes the front surface side of the compartment S, and the rear block 48B constitutes the rear surface side of the compartment S.

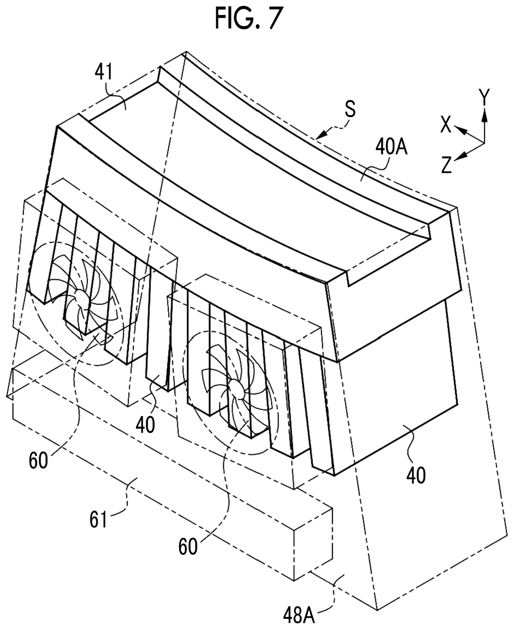

As shown in FIG. 7, one compartment S includes a plurality of detector modules 40. The number of detector modules 40 included in the compartment S is not limited, and at least one detector module 40 may be included in the compartment S. The compartment S is a unit of intake and exhaust for heat dissipation, which will be described in detail below. The plurality of partition plates 48D separate intake and exhaust for each compartment S.

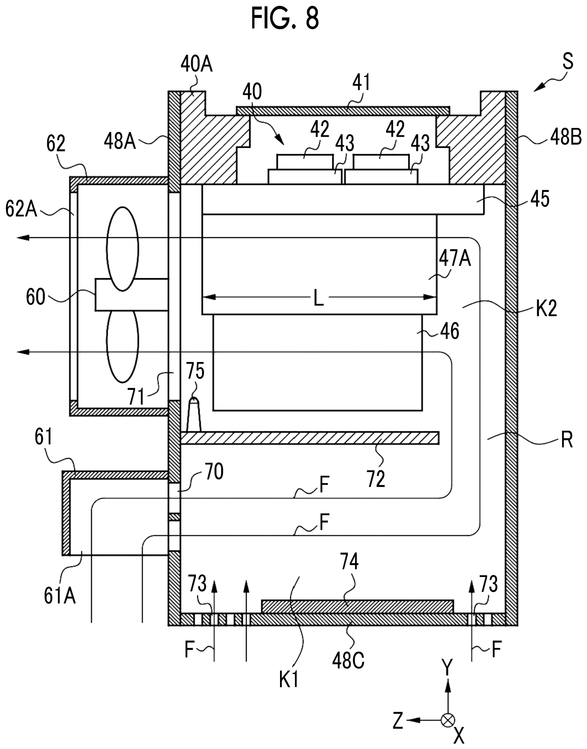

FIG. 8 shows an internal configuration of the compartment S. The compartment S has at least one intake port 70 and at least one exhaust port 71. In the present embodiment, the compartment S has one intake port 70 and two exhaust ports 71. Each of the intake port 70 and the exhaust port 71 is formed in the front cover 48A. That is, the intake port 70 and the exhaust port 71 are disposed on the same side (the front surface side in the present embodiment) with respect to the heat sink 47. The intake port 70 and the exhaust port 71 are disposed at positions at different distances from the central axis A of the rotating plate 50. Specifically, the intake port 70 is disposed at a position that is farther from the central axis A than the exhaust port 71. The distances of the two exhaust ports 71 from the central axis A are equal to each other.

In addition, an intake cover 61 that surrounds the periphery of the intake port 70 is provided in the front cover 48A. An opening 61A for air intake is formed in the intake cover 61. The intake cover 61 has, for example, a rectangular parallelepiped shape with one open side. The opening 61A is formed on a side opposite to the exhaust port 71 (that is, a side far from the central axis A) such that the air exhausted from the exhaust port 71 is not drawn in. The intake port 70 and the intake cover 61 extend in the X direction to span the two exhaust ports 71. In addition, one intake port 70 may be provided for one exhaust port 71. That is, one intake cover 61 may be provided for one exhaust cover 62. The intake cover 61 and the exhaust cover 62 function as separation plates that separate intake and exhaust outside the compartment S.

The exhaust fan 60 is attached to the exhaust port 71. The exhaust fan 60 is attached to the outside of the front cover 48A and exhausts air through the exhaust port 71. In addition, the exhaust cover 62 that surrounds the periphery of the exhaust port 71 is provided in the front cover 48A. The exhaust cover 62 also covers the periphery of the exhaust fan 60. The shape of the exhaust cover 62 is not limited and may be a cylindrical shape having the rotation axis of the exhaust fan 60 as the center. An opening 62A for air exhaust is formed in the exhaust cover 62. The opening 62A may be covered with a mesh-like member or the like as long as air can pass through the opening 62A.

In addition, a separation plate 72 for separating intake and exhaust inside the compartment S is provided between the intake port 70 and the exhaust port 71. In the present embodiment, the separation plate 72 has one end connected to the front cover 48A and the other end extending toward the rear block 48B. The front cover 48A and the rear block 48B are spaced apart from each other and constitute a communication portion R. The separation plate 72 separates the inside of the compartment S into a space K1 into which air flows from the intake port 70 and a space K2 from which air flows out toward the exhaust port 71. The space K1 and the space K2 communicate with each other through the communication portion R.

The plurality of fins 47A of the heat sink 47 extend along the flow path of the air exhausted from the exhaust port 71. A groove that is formed by two adjacent fins 47A and extends in the Z direction is formed to follow the flow path of the air that is drawn in or exhausted.

In addition, a plurality of through-holes 73 communicating with the outside are formed in the bottom cover 48C. The through-hole 73 is an additional intake port for additionally drawing air into the internal space K1 from the outside of the compartment S. The through-hole 73 is formed in a region that is not irradiated with the X-rays, which have been emitted from the X-ray source 3 and transmitted through the detector module 40, in the bottom cover 48C. A shielding plate 74 that contains lead and blocks the X-rays is provided in a region that is irradiated with the X-rays in the bottom cover 48C.

At least one temperature measurement device 75 is provided inside the compartment S. The temperature measurement device 75 is, for example, a thermistor and is disposed between the exhaust fan 60 and the detector module 40 to correspond to each exhaust port 71. For example, the temperature measurement device 75 is disposed on the separation plate 72. The temperature measurement device 75 is connected to the control substrate 80 through a signal line (not shown). The temperature measurement device 75 measures the temperature (hereinafter, referred to as an exhaust temperature) of the air exhausted from the exhaust port 71 by the exhaust fan 60 and transmits the measured value of the temperature to the control substrate 80.

The exhaust fan 60 is connected to the control substrate 80 through a signal line (not shown). The control substrate 80 adjusts the rotation speed of the exhaust fan 60 based on the measured value of the exhaust temperature. In the present disclosure, the rotation speed refers to the number of rotations per unit time.

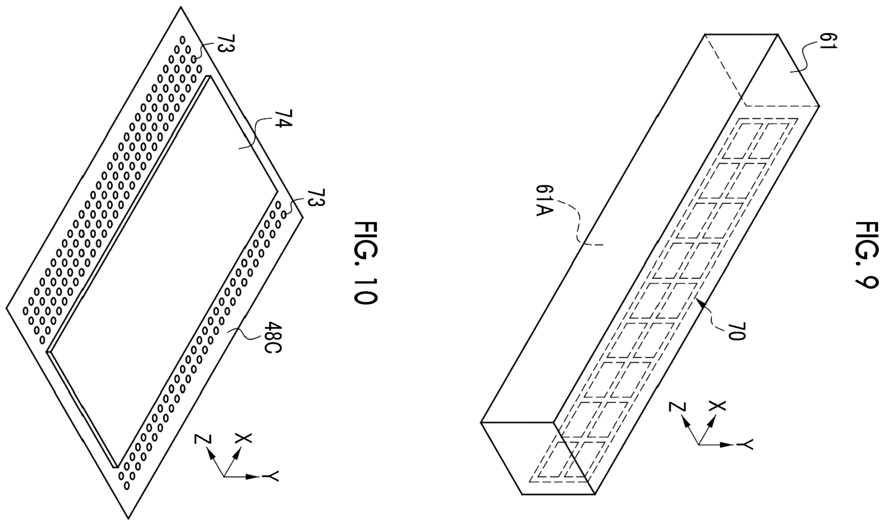

FIG. 9 shows a configuration of the intake port 70. The intake port 70 has a lattice-like opening shape. This is intended for electromagnetic interference (EMC) countermeasures. Specifically, a shielding effect of preventing the entry of unnecessary electromagnetic waves from the outside, suppressing the leakage of electromagnetic waves generated from the detector module 40 in the compartment S, and reducing the influence on surrounding devices can be obtained.

FIG. 10 shows a bottom portion of the compartment S. The plurality of through-holes 73 are disposed on both sides of the region, in which the shielding plate 74 is provided, in the Z direction in the bottom cover 48C. This makes it possible to ensure an appropriate ventilation path while maintaining the radiation shielding effect.

Next, the temperature control of the X-ray detection device 4 by the control substrate 80 will be described. The main causes of the temperature rise of the X-ray detection device 4 include the generation of heat in the ASIC 43 due to photon counting, a rise in the internal temperature of the scanner due to X-ray exposure, and a change in the heat distribution of the scanner.

Specifically, in the CT apparatus 2, since the photons of the X-rays are individually counted, the calculation load on the photon counting circuit 44 increases, and the amount of heat generated increases. In particular, since the power consumption of the ASIC 43 varies depending on the amount of X-ray attenuation of the subject H, the distribution of the exhaust temperature differs depending on the position in the X direction. In addition to the temperature rise due to the heat generation, the amount of heat generated in the X-ray tube 31 is also increased by irradiation with the X-rays, and the internal temperature of the scanner rises due to the influence of the increase in the amount of heat generated. As a result, the intake temperature rises, and the temperature of the detector module 40 rises. Further, the heat distribution changes depending on the state of the scanner. In a case where the scanner is stationary, the heat of the X-ray tube 31 is mainly dissipated from the upper portion of the scanner to the outside of the scanner. However, in a case where the scanner is rotated, the heat is diffused throughout the inside of the scanner, which affects the intake temperature. Since these temperature changes cause the temperatures of the plurality of detector modules 40 to be non-uniform, it is necessary to efficiently perform temperature control with high accuracy.

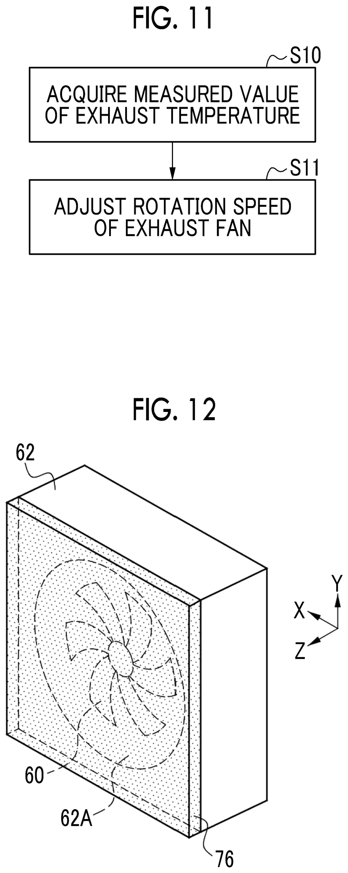

FIG. 11 shows the flow of the temperature control by the control substrate 80. For example, the control substrate 80 acquires the measured value of the exhaust temperature from each temperature measurement device 75 during scanning preparation (Step S10). Then, the control substrate 80 adjusts the rotation speed of each exhaust fan 60 based on the acquired measured values (Step S11). Specifically, the control substrate 80 increases the rotation speed of the exhaust fan 60 as the exhaust temperature increases. The reason is that, as the rotation speed of the exhaust fan 60 increases, the amount of exhaust air increases and the cooling effect is improved.

For example, the control substrate 80 constantly monitors the exhaust temperature and controls the rotation speed of the exhaust fan 60. In this case, the control substrate 80 repeatedly executes Step S10 and Step S11. In addition, the control substrate 80 may monitor the exhaust temperature before the start of scanning and then control the rotation speed of the exhaust fan 60 to perform temperature control. In this case, the control substrate 80 executes Step S10 and then repeatedly executes Step S11.

Letter F shown in FIG. 8 schematically indicates the flow of air that is drawn in and exhausted. As the exhaust fan 60 is rotated, the air drawn in through the intake port 70 flows from the space K1 into the space K2 through the communication portion R. In the space K2, the air flows toward the exhaust port 71. A portion of the flow of the air is cooled as the air loses heat while passing through the heat sink 47. Then, the air is exhausted to the outside through the exhaust port 71.

In the technology of the present disclosure, cooling is performed only using the exhaust fan without providing the intake fan unlike the related art and the rotation speed of the exhaust fan is controlled based on the exhaust temperature. Therefore, it is possible to control the temperature with high accuracy while suppressing the complexity of the structure.

In the related art, the intake fan, the exhaust fan, and the opening and closing mechanism are combined to perform cooling. However, the use of the intake fan has the problem that cooling air is likely to concentrate on a portion of the detector module, resulting in temperature variations. In the technology of the present disclosure, only the exhaust fan is used, and natural intake is performed by negative pressure. Therefore, it is possible to create a uniform air flow in the entire region in which the detector module is disposed and to prevent local cooling insufficiency.

In addition, in the technology of the present disclosure, since the intake fan and the opening and closing mechanism are not necessary, the structure of the apparatus is simplified, and the number of components is reduced. Therefore, it is possible to reduce costs and to improve maintainability.

Further, in the technology of the present disclosure, since the rotation speed of the exhaust fan is adjusted based on the exhaust temperature, it is possible to understand the actual thermal state of the detector module in real time and to perform appropriate temperature control. It is possible to promptly respond to temperature variations caused by a plurality of factors, such as fluctuations in heat generation in the ASIC due to photon counting and temperature rise inside the scanner, and to stably maintain the temperature of the detector module.

Furthermore, since the exhaust temperature is an index that comprehensively reflects the cooling state in the compartment, it is possible to prevent local undercooling or overheating and to suppress temperature variations.

In the technology of the present disclosure, the plate-fin-type heat sink is used in order to improve the cooling efficiency of the detector module.

In general, the heat sink has a shape, such as a plate fin type or a pin type, and the cooling performance varies depending on each shape. The plate-fin-type heat sink has a structure in which a plurality of parallel fins are arranged at regular intervals, and the flow of air is likely to be rectified along the fins. As a result, the volume of the air passing through the heat sink is ensured, and it is possible to efficiently dissipate heat. On the other hand, the pin-type heat sink has a structure in which a plurality of columnar pins are arranged. However, the flow of air is likely to be disturbed, and the cooling effect tends to decrease.

In the technology of the present disclosure, since the plate-fin-type heat sink is used, it is possible to efficiently use the flow of air generated by the exhaust fan and to uniformly cool the entire heat sink. As a result, the uniformity of the heat distribution in each portion of the heat sink is improved, which makes it possible to suppress local temperature rise. In addition, in order to improve the cooling efficiency, it is preferable to determine the arrangement pitch of the fins based on a balance between the surface area and the volume of air passing through the heat sink.

In addition, in the technology of the present disclosure, the intake port and the exhaust port are disposed on the same side (the front surface side in the above-described embodiment) with respect to the heat sink, and the heat sink has a plurality of fins that extend along the flow path of the air exhausted from the exhaust port. Therefore, the problem that the amount of intake and exhaust air is reduced due to the structure on the rear surface side, resulting in a reduction in cooling efficiency, as in a case where the intake port or the exhaust port is provided on the rear surface side is suppressed. That is, according to the technology of the present disclosure, it is possible to efficiently perform cooling while suppressing the complexity of the structure.

Hereinafter, various modification examples of the above-described embodiment will be described.

### First Modification Example

FIG. 12 shows an exhaust cover 62 according to a first modification example. In the present modification example, the opening 62A of the exhaust cover 62 is covered with a sound absorbing cover 76 in order to reduce noise generated from the exhaust fan 60.

In a case where the exhaust fan 60 is operated, noise is generated due to turbulence caused by the flow of air or the vibration of the exhaust fan 60. In particular, a noise level increases during high-speed rotation. Since the opening 62A of the exhaust cover 62 is covered with the sound absorbing cover 76, it is possible to effectively absorb the noise radiated from the opening 62A and to suppress the propagation of sound to the outside. It is preferable to use a porous material, a fiber-based material, or the like as a material forming the sound absorbing cover 76. In addition, the opening 61A of the intake cover 61 may be covered with a sound absorbing cover in order to suppress noise radiated from the intake port 70.

### Second Modification Example

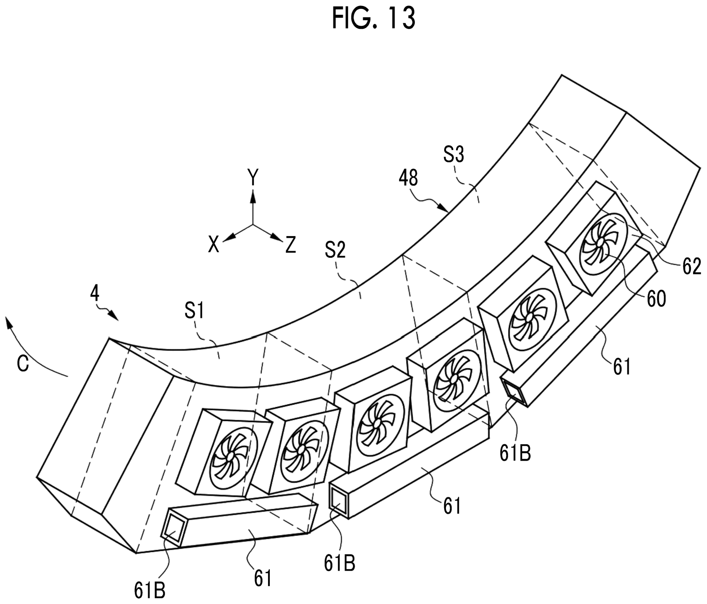

FIG. 13 shows an X-ray detection device 4 according to a second modification example. In the present modification example, an opening 61B is provided in the intake cover 61 on the side of the rotation direction C of the scanner in order to efficiently draw air into the X-ray detection device 4 through the intake port 70.

In a case where the scanner is rotated, external air flows along the rotation direction. Therefore, the provision of the opening 61B in the intake cover 61 on the side of the rotation direction C makes it possible to promote natural intake using the rotating airflow. As a result, efficient intake can be achieved by combining the negative pressure generated by the exhaust fan 60 and the rotating airflow of the scanner, without using the intake fan. In addition, it is preferable that the shape and size of the opening 61B are determined to optimize the flow of intake air.

### Third Modification Example

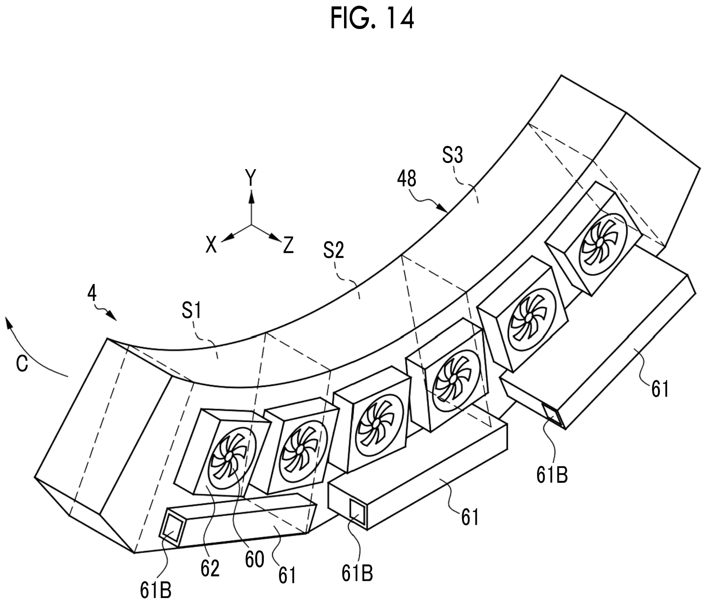

FIG. 14 shows an X-ray detection device 4 according to a third modification example. In the present modification example, the lengths of a plurality of intake covers 61 in the Z direction are changed in addition to the configuration of the second modification example. Specifically, the lengths of the intake covers 61 in the Z direction are reduced stepwise toward the rotation direction C of the scanner. Specifically, in the present modification example, in a case where the length of the intake cover 61 of the compartment S1 in the Z direction is represented by LA1, the length of the intake cover 61 of the compartment S2 in the Z direction is represented by LA2, and the length of the intake cover 61 of the compartment S3 in the Z direction is represented by LA3, a relationship of LA1 < LA2 < LA3 is satisfied.

In order to achieve efficient intake using the airflow generated with the rotation of the scanner, it is important to adjust the flow of air toward the intake port 70 and to reduce factors that obstruct the airflow. In particular, the intake cover 61 located on the side of the rotation direction C may obstruct the airflow. Therefore, in the present modification example, the lengths of the plurality of intake covers in the Z direction are reduced stepwise toward the rotation direction C to adjust the flow of air and thus to achieve smooth intake.

### Fourth Modification Example

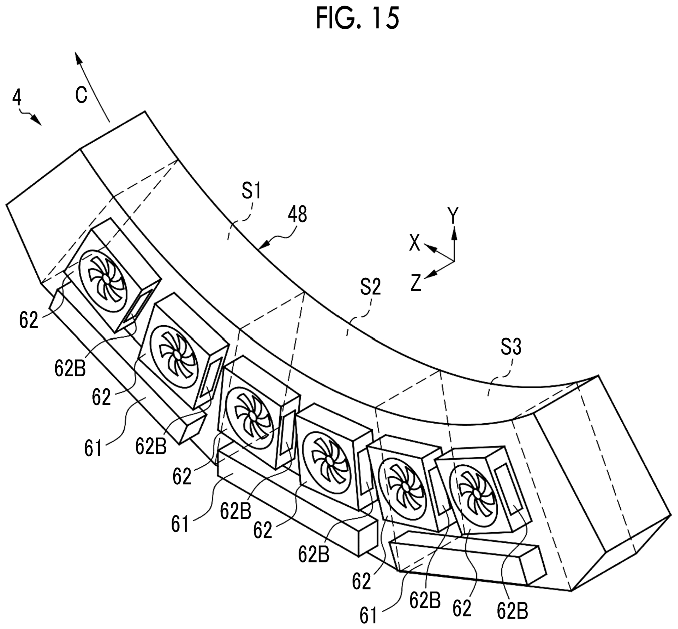

FIG. 15 shows an X-ray detection device 4 according to a fourth modification example. In the present modification example, an opening 62B is provided on a side of the exhaust cover 62 opposite to the rotation direction C of the scanner in order to promote exhaust and improve cooling efficiency.

In a case where the scanner is rotated, the air inside the exhaust cover 62 also flows along the rotation direction C. Therefore, the exhaust efficiency is improved by providing the opening 62B on the side of the exhaust cover 62 opposite to the rotation direction C. In addition, it is preferable that the shape and size of the opening 62B are determined to optimize the flow of the air that is exhausted.

Further, the present modification example may be applied to the X-ray detection device 4 according to the second modification example or the third modification example.

### Fifth Modification Example

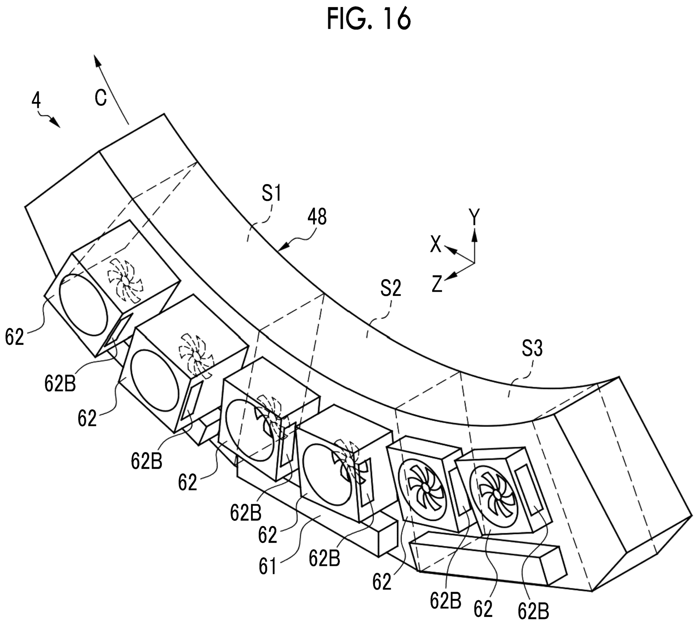

FIG. 16 shows an X-ray detection device 4 according to a fifth modification example. In the present modification example, the lengths of the plurality of exhaust covers 62 in the Z direction are changed in addition to the configuration of the fourth modification example. Specifically, the lengths of the exhaust covers 62 in the Z direction are reduced stepwise toward the side opposite to the rotation direction C of the scanner. Specifically, in the present modification example, in a case where the length of the exhaust cover 62 of the compartment S1 in the Z direction is represented by LB1, the length of the exhaust cover 62 of the compartment S2 in the Z direction is represented by LB2, and the length of the exhaust cover 62 of the compartment S3 in the Z direction is represented by LB3, a relationship of LB1 > LB2 > LB3 is satisfied.

In order to efficiently exhaust air using the airflow generated with the rotation of the scanner, it is important to adjust the flow of air from the exhaust port and to reduce factors that obstruct the airflow. Therefore, in the present modification example, the lengths of the plurality of exhaust covers 62 in the Z direction are reduced toward the side opposite to the rotation direction C to adjust the flow of air and to achieve smooth exhaust.

In addition, in the present modification example, the lengths of two exhaust covers 62 of one compartment S in the Z direction are equal to each other. However, for the lengths of the two exhaust covers 62 in the Z direction, the length of the exhaust cover 62 on the side opposite to the rotation direction C may be reduced. Further, the present modification example may be applied to the X-ray detection device 4 according to the second modification example or the third modification example.

### Sixth Modification Example

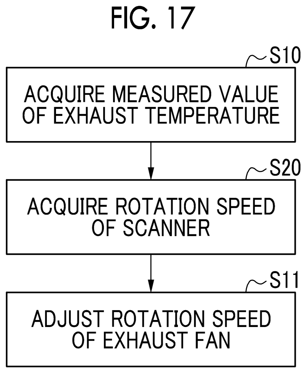

FIG. 17 shows a flow of temperature control according to a sixth modification example. In the present modification example, the control substrate 80 acquires the rotation speed of the scanner in Step S20 after Step S10. In addition, in the present modification example, after Step S20, the control substrate 80 adjusts the rotation speed of each exhaust fan 60 based on the acquired measured value and rotation speed in Step S11. Specifically, the control substrate 80 increases the rotation speed of the exhaust fan 60 as the exhaust temperature increases and decreases the rotation speed of the exhaust fan 60 as the rotation speed of the scanner increases.

In a case where the scanner is rotated at a high speed, the flow of air is strong due to the rotation. Therefore, noise can be reduced by suppressing the rotation speed of the exhaust fan 60. On the other hand, in a case where the scanner is rotated at a low speed or stopped, the exhaust is insufficient. Therefore, the rotation speed of the exhaust fan 60 can be increased to maintain appropriate heat dissipation.

Further, in the above-described embodiment, the technology of the present disclosure has been described using the PCCT-type CT apparatus. However, the present disclosure is not limited thereto and can also be applied to an energy-integrating CT apparatus according to the related art.

Even in the energy-integrating CT apparatus, heat is generated from the X-ray tube as a result of irradiation with the X-rays. Therefore, the cooling and temperature control of the X-ray detection device are important issues as in the PCCT type. In the present disclosure, cooling techniques, such as the disposition of the intake port and the exhaust port, the control of the rotation speed of the exhaust fan based on the exhaust temperature, dynamic exhaust adjustment considering the rotation speed of the scanner, the optimization of the fin structure of the heat sink, and the optimization of the configurations of the intake cover and the exhaust cover, can be combined to achieve efficient cooling and highly accurate temperature control even in the energy-integrating CT apparatus.

In addition, in the above-described embodiment, the temperature measurement device 75 is provided on the separation plate 72. However, the temperature measurement device 75 may be incorporated into each ASIC 43. In this case, the control substrate 80 adjusts the rotation speed of the exhaust fan 60 based on the measured value of the temperature by the temperature measurement device 75 incorporated into each ASIC 43 included in the compartment S. For example, the control substrate 80 adjusts the rotation speed of the exhaust fan 60 based on the average value, median value, or the like of a plurality of measured temperature values measured by a plurality of temperature measurement devices 75 in the compartment S.

In addition, in each of the above-described embodiment, the X-rays have been described as an example of the radiation. However, γ-rays may be used as the radiation.

Further, in the above-described embodiment, various processors described below can also be used as the configuration related to the control of the control substrate 80. The various processors include a CPU, which is a general-purpose processor that executes software (programs) to function as various processing units, a programmable logic device (PLD) whose circuit configuration can be changed after manufacturing, such as a field-programmable gate array (FPGA), and a dedicated electric circuit which is a processor having a circuit configuration dedicatedly designed for executing a specific process such as an ASIC.

In addition, the above-described various processes may be executed using one of these various processors or may be executed using a combination of two or more processors of the same type or different types (for example, a plurality of FPGAs, a combination of a CPU and an FPGA, and the like). Alternatively, a plurality of processing units may be configured by one processor. An example in which a plurality of processing units are configured by one processor is an aspect in which a processor that implements all functions of a system including a plurality of processing units with one integrated circuit (IC) chip is used, as in a system on a chip (SOC) and the like.

It is possible to understand the techniques described in the following supplementary notes from the above description.

### Supplementary Note 1

A radiation detection device that is divided into a plurality of compartments, the radiation detection device comprising in each of the plurality of compartments:
a detector module that detects radiation;
a heat sink that is attached to the detector module;
an intake port and an exhaust port that are disposed on the same side with respect to the heat sink;
a separation plate that is provided between the intake port and the exhaust port and separates intake and exhaust; and
an exhaust fan that is attached to the exhaust port,
in which the heat sink has a plurality of fins that extend along a flow path of air exhausted from the exhaust port.

### Supplementary Note 2

A CT apparatus comprising:
a radiation source that emits radiation; and
a radiation detection device that is divided into a plurality of compartments,
in which the radiation source and the radiation detection device are configured to be rotatable about a central axis in a state in which the radiation source and the radiation detection device face each other,
the CT apparatus includes, in each of the plurality of compartments,
   a detector module that detects the radiation,
   a heat sink that is attached to the detector module,
   an intake port and an exhaust port that are disposed on the same side with respect to the heat sink,
   a separation plate that is provided between the intake port and the exhaust port and separates intake and exhaust, and
   an exhaust fan that is attached to the exhaust port, and
the heat sink has a plurality of fins that extend along a flow path of air exhausted from the exhaust port.

### Supplementary Note 3

The CT apparatus according to Supplementary Note 2,
in which the plurality of fins form a groove in a direction along the flow path.

### Supplementary Note 4

The CT apparatus according to Supplementary Note 2 or 3,
in which the intake port and the exhaust port are formed in a front cover that constitutes a front surface side of each of the plurality of compartments.

### Supplementary Note 5

The CT apparatus according to any one of Supplementary Notes 2 to 4,
in which the intake port and the exhaust port are disposed at different distances from the central axis.

### Supplementary Note 6

The CT apparatus according to Supplementary Note 5,
in which the intake port is disposed at a position that is farther from the central axis than the exhaust port.

### Supplementary Note 7

The CT apparatus according to any one of Supplementary Notes 2 to 6,
in which the plurality of compartments are partitioned by a plurality of partition plates.

### Supplementary Note 8

The CT apparatus according to any one of Supplementary Notes 2 to 7,
in which an intake cover that covers a periphery of the intake port and an exhaust cover that covers a periphery of the exhaust port are provided in each of the plurality of compartments, and
intake and exhaust are separated from each other outside the compartment by the intake cover and the exhaust cover.

### Supplementary Note 9

The CT apparatus according to Supplementary Note 8,
in which an opening is formed in the intake cover on a side of a rotation direction in which the radiation source and the radiation detection device are rotated.

### Supplementary Note 10

The CT apparatus according to Supplementary Note 9,
in which lengths of a plurality of the intake covers in a direction of the central axis are reduced stepwise toward the rotation direction.

### Supplementary Note 11

The CT apparatus according to any one of Supplementary Notes 8 to 10,
in which an opening is formed in the exhaust cover on a side opposite to the rotation direction in which the radiation source and the radiation detection device are rotated.

### Supplementary Note 12

The CT apparatus according to Supplementary Note 11,
in which the lengths of a plurality of the exhaust covers in the direction of the central axis are reduced stepwise toward the side opposite to the rotation direction.

### Supplementary Note 13

The CT apparatus according to any one of Supplementary Notes 2 to 12,
in which the intake port has a lattice-like opening shape.

### Supplementary Note 14

The CT apparatus according to any one of Supplementary Notes 2 to 13,
in which a plurality of through-holes and a shielding plate that shields the radiation are provided in a bottom portion of each of the plurality of compartments, and
the shielding plate is disposed in a region that is irradiated with the radiation transmitted through the detector module.

### Supplementary Note 15

The CT apparatus according to any one of Supplementary Notes 8 to 12,
in which a sound absorbing sheet is attached to at least one of the intake cover or the exhaust cover.

### Supplementary Note 16

The CT apparatus according to any one of Supplementary Notes 2 to 15, further comprising:
a temperature measurement device that measures a temperature of the air exhausted from the exhaust port; and
a control substrate that controls a rotation speed of the exhaust fan based on the temperature.

### Supplementary Note 17

The CT apparatus according to Supplementary Note 16,
in which the temperature measurement device is disposed between the detector module and the exhaust fan.

### Supplementary Note 18

The CT apparatus according to Supplementary Note 16 or 17,
in which the control substrate controls the rotation speed of the exhaust fan based on a rotation speed of the radiation source and the radiation detection device, in addition to the temperature.

### Supplementary Note 19

The CT apparatus according to Supplementary Note 16,
in which the detector module includes a circuit element including a photon counting circuit that counts the number of photons, and
the temperature measurement device is incorporated into the circuit element.

### Supplementary Note 20

The CT apparatus according to Supplementary Note 19,
in which the detector module includes a plurality of the circuit elements,
the temperature measurement device is incorporated into each of the plurality of circuit elements, and
the control substrate controls the rotation speed based on an average value or a median value of a plurality of measured temperature values.

### Explanation of References

2: CT apparatus
3: X-ray source
4: X-ray detection device
5: gantry
5A: opening portion
6: bed
7: controller
8: image processing unit
9: input device
10: display device
11: storage device
12: communication device
31: X-ray tube
32: X-ray filter
33: bowtie filter
40: detector module
40A: case
41: collimator
42: semiconductor layer
43: ASIC
44: photon counting circuit
45: frame
46: circuit substrate
47: heat sink
47A: fin
48: housing
48A: front cover
48B: rear block
48C: bottom cover
48D: partition plate
49: substrate
50: rotating plate
51: bearing
52: frame
53: slip ring
54: fixing portion
60: exhaust fan
61: intake cover
61A, 61B: opening
62: exhaust cover
62A, 62B: opening
70: intake port
71: exhaust port
72: separation plate
73: through-hole
74: shielding plate
75: temperature measurement device
76: sound absorbing cover
80: control substrate
A: central axis
C: rotation direction
H: subject
K1, K2: space
R: communication portion
S, S1 to S3: compartment

## Claims

1. A radiation detection device that is divided into a plurality of compartments, the radiation detection device comprising in each of the plurality of compartments:
a detector module that detects radiation;
a heat sink that is attached to the detector module;
an intake port and an exhaust port that are disposed on the same side with respect to the heat sink;
a separation plate that is provided between the intake port and the exhaust port and separates intake and exhaust; and
an exhaust fan that is attached to the exhaust port,
wherein the heat sink has a plurality of fins that extend along a flow path of air exhausted from the exhaust port.

2. ACT apparatus comprising:
a radiation source that emits radiation; and
a radiation detection device that is divided into a plurality of compartments,
wherein the radiation source and the radiation detection device are configured to be rotatable about a central axis in a state in which the radiation source and the radiation detection device face each other,
the CT apparatus includes, in each of the plurality of compartments,
a detector module that detects the radiation,
a heat sink that is attached to the detector module,
an intake port and an exhaust port that are disposed on the same side with respect to the heat sink,
a separation plate that is provided between the intake port and the exhaust port and separates intake and exhaust, and
an exhaust fan that is attached to the exhaust port, and
the heat sink has a plurality of fins that extend along a flow path of air exhausted from the exhaust port.

3. The CT apparatus according to claim 2,
wherein the plurality of fins form a groove in a direction along the flow path.

4. The CT apparatus according to claim 2 or 3,
wherein the intake port and the exhaust port are formed in a front cover that constitutes a front surface side of each of the plurality of compartments.

5. The CT apparatus according to any one of claims 2 to 4,
wherein the intake port and the exhaust port are disposed at different distances from the central axis.

6. The CT apparatus according to claim 5,
wherein the intake port is disposed at a position that is farther from the central axis than the exhaust port.

7. The CT apparatus according to any one of claims 2 to 6,
wherein the plurality of compartments are partitioned by a plurality of partition plates.

8. The CT apparatus according to any one of claims 2 to 7,
wherein an intake cover that covers a periphery of the intake port and an exhaust cover that covers a periphery of the exhaust port are provided in each of the plurality of compartments, and
intake and exhaust are separated from each other outside the compartment by the intake cover and the exhaust cover.

9. The CT apparatus according to claim 8,
wherein an opening is formed in the intake cover on a side of a rotation direction in which the radiation source and the radiation detection device are rotated.

10. The CT apparatus according to claim 9,
wherein lengths of a plurality of the intake covers in a direction of the central axis are reduced stepwise toward the rotation direction.

11. The CT apparatus according to any one of claims 8 to 10,
wherein an opening is formed in the exhaust cover on a side opposite to a rotation direction in which the radiation source and the radiation detection device are rotated.

12. The CT apparatus according to claim 11,
wherein lengths of a plurality of the exhaust covers in a direction of the central axis are reduced stepwise toward the side opposite to the rotation direction.

13. The CT apparatus according to any one of claims 2 to 12,
wherein the intake port has a lattice-like opening shape.

14. The CT apparatus according to any one of claims 2 to 13,
wherein a plurality of through-holes and a shielding plate that shields the radiation are provided in a bottom portion of each of the plurality of compartments, and
the shielding plate is disposed in a region that is irradiated with the radiation transmitted through the detector module.

15. The CT apparatus according to any one of claims 8 to 14,
wherein a sound absorbing sheet is attached to at least one of the intake cover or the exhaust cover.

16. The CT apparatus according to any one of claims 2 to 15, further comprising:
a temperature measurement device that measures a temperature of the air exhausted from the exhaust port; and
a control substrate that controls a rotation speed of the exhaust fan based on the temperature.

17. The CT apparatus according to claim 16,
wherein the temperature measurement device is disposed between the detector module and the exhaust fan.

18. The CT apparatus according to claim 16 or 17,
wherein the control substrate controls the rotation speed of the exhaust fan based on a rotation speed of the radiation source and the radiation detection device, in addition to the temperature.

19. The CT apparatus according to any one of claims 16 to 18,
wherein the detector module includes a circuit element including a photon counting circuit that counts the number of photons, and
the temperature measurement device is incorporated into the circuit element.

20. The CT apparatus according to claim 19,
wherein the detector module includes a plurality of the circuit elements,
the temperature measurement device is incorporated into each of the plurality of circuit elements, and
the control substrate controls the rotation speed based on an average value or a median value of a plurality of measured temperature values.
